# EUROPEAN PATENT SPECIFICATION

(11) **EP 1 521 846 B1**
(45) Date of publication and mention of the grant of the patent: **06.03.2013**
(21) Application number: 03740789.7
(22) Date of filing: 11.07.2003
(51) Int. Cl.: C12Q 1/68, G01N 21/65, G01N 33/543, G01N 33/531, B01J 13/14

(54) **SERRS REACTIVE PARTICLES**
SERRS REAKTIVE TEILCHEN
PARTICULES REACTIVES SERRS

(30) Priority: 12.07.2002 GB 0216197
(43) Date of publication of application: 13.04.2005
(73) Proprietor: University of Strathclyde, Glasgow G1 1XQ (GB)
(72) Inventor: SMITH, William Ewen, Glasgow G61 3BD (GB); GRAHAM, Duncan, Corstorphine Edinburgh EH12 7LQ (GB); CORMACK, Peter University of Strathclyde, 295 Cathedral Street Glasgow G1 1XL (GB); McCABE, Ailie University of Strathclyde, 295 Cathedral Street Glasgow G1 1XL (GB)
(74) Representative: Chapman, Paul Gilmour
(86) International application number: PCT/GB2003/003009
(87) International publication number: WO 2004/007767

(56) References cited:
- EP-A- 0 806 460
- WO-A-97/05280
- WO-A-99/60157
- GB-A- 1 403 197
- US-A- 5 721 102
- RODGER C ET AL: "Surface-enhanced resonance-Raman scattering: an informative probe of surfaces" JOURNAL OF THE CHEMICAL SOCIETY, DALTON TRANSACTIONS, CHEMICAL SOCIETY. LETCHWORTH, GB, 1996, pages 791-799, XP002106902 ISSN: 1472-7773 cited in the application
- MUNRO C H ET AL: "QUALITATIVE AND SEMI-QUANTITATIVE TRACE ANALYSIS OF ACIDIC MONOAZO DYES BY SURFACE ENHANCED RESONANCE RAMAN SCATTERING" ANALYST, LONDON, GB, vol. 120, no. 4, 1 April 1995 (1995-04-01), pages 993-1003, XP000196569
- WOOLLEY P S ET AL: "Surface-enhanced resonance Raman spectra of water-insoluble tetraphenylporphyrin and chlorophyll a on silver hydrosols with a dioxane molecular spacer" CHEMICAL PHYSICS LETTERS, 16 AUG. 1996, ELSEVIER, NETHERLANDS, vol. 258, no. 3-4, pages 501-506, XP002263622 ISSN: 0009-2614
- MUNRO C.H. ET AL.: "Characterization of the surface of a citrate-reduced colloid optimized for use as a substrate for surface-enhanced resonance Raman scattering" LANGMUIR, vol. 11, 1995, pages 3712-3720, XP002263598 cited in the application
- LEE P C ET AL: "ADSORPTION AND SURFACE-ENHANCED RAMAN OF DYES ON SILVER AND GOLD SOLS" JOURNAL OF PHYSICAL CHEMISTRY, AMERICAN CHEMICAL SOCIETY, US, vol. 86, no. 17, 19 August 1982 (1982-08-19), pages 3391-3395, XP001118070 ISSN: 0022-3654 cited in the application

## Description

The present invention relates to the provision of SERRS active polymer beads and a method of their production for use in detecting target molecules, as well as methods of detecting target molecules.

Recent developments in modern molecular biology have highlighted the need for simultaneous *in-situ* recognition of a large number of bioanalytes. Among the fields for which there is a requirement is DNA detection, proteomics, cell and antibody recognition, and drug discovery.

Related technologies which involve particles and use detection techniques other than SERRS, include the use of "quantum dots" and the use of the change in surface plasmon resonance which can be achieved by association of gold particles. Quantum dots consist of nanoscale particles of cadmium sulphide/selenide, a well known pigment, which can be modified so that a bioanalyte can be attached. The advantage of this technique is that a single particle gives intense emission compared to a single molecule and the nature of the particles is such that a wide range of emission frequencies can be achieved by altering the ratio of sulphide to selenide.

In the surface plasmon resonance approach, as described in Storhoff, J.J., Elghanian, R., Mucic, R.C., Mirkin, C.A., Letsinger, R.L., J. Am. Chem. Soc 1998, 120, 1959-1964 two gold nanoparticles modified on the surface with specific oligonucleotides are used. Both particles recognise and hybridise a specific strand of DNA which is the analyte. This brings the gold particles into close proximity and the change in frequency of the surface plasmon resonance is detected. However, such techniques do not offer the combined multiplexing, robustness and simplicity which would be desired.

SERRS can uniquely provide the selective labelling chemistry to develop the new types of assays required. It has a sensitivity equivalent to or better than fluorescence but the key advantage is that each SERRS dye used as a label gives a unique fingerprint, which can be recognized in a dye mixture without separation. There are now about 50 specially designed dyes for SERRS, each of which gives a unique spectrum. Although effective methods for harnessing this potential are known, the development is limited by two problems. Firstly there is the possibility of a reaction between the dye coated silver particles and the analytical reagents they label or other reagents and, secondly, to obtain stable intense SERRS it is preferable to maintain and reproduce a specific degree of aggregation among the silver particles - a procedure which can easily be upset during the analysis.

WO 97/05280 A discloses an association of a label-containing complex with a SERRS-active surface, wherein said association comprises an aggregated metal colloid and a SERRS active dye chemisorbed thereto. The aggregated metal colloid may be coated with an organic polymer.

It is amongst the objectives of the present invention to obviate and/or mitigate at least one of the aforementioned disadvantages.

Generally speaking, the present invention is based on observations by the present inventors that a SERRS active dye can be encapsulated within a polymer coating, conferring stability to the dye, protecting the SERRS active dye from the environment and allowing more than one dye to be encapsulated such that more than one SERRS signal can be detected.

Thus, in a first aspect the present invention provides a SERRS active bead for use in identification of a target molecule, wherein the SERRS active bead comprises aggregated metal colloid and at least one SERRS active dye, encapsulated within a polymer shell.

SERRS refers to surface enhanced resonance Raman scattering. SERRS has been previously described, see for example Rodger, C.H., Smith, W.E., Dent, G., Edmonson, M., J. Chem. Soc. Dalton Trans., 1996, 5, 791 and references therein to which the reader is directed for background information. Surprisingly, there is a widespread fluorescence quenching mechanism on the silver surface. This means that almost all dyes give SERRS enabling the use of more extensive and simpler derivatisation chemistry than is possible by fluorescence. Further the specific enhancement of the dye provided by SERRS active particles is more than sufficient to allow the identification of the dye from the background signals normally observed from biological and industrial samples containing the target analyte. This eliminates the need for complex time consuming separation procedures required with many techniques before the analyte can be successfully identified.

It is to be appreciated that the term SERRS as used herein should not be construed as limiting and the present invention may also be applied to surface enhanced Raman scuttering (SERS), but SERRS is preferred.

Surprisingly, it has been found that encapsulating the SERRS active, metal nanoparticle and SERRS active dye within a polymer coating, does not prevent the SERRS signal from being easily detected. Moreover, the SERRS signal can be detected at a distance, in certain circumstances, of up to 10 metres from the beads.

The polymer shell may be formed from a wide variety of monomers and cross linkers including monomers that polymerise via chain-growth mechanisms and step-growth mechanisms capable of forming a bead which encapsulates the aggregated colloid and SERRS active dye. It is understood that a portion of the aggregated colloid may also be attached and/or embedded on the surface of the bead(s). The polymer shell may comprise more than one layer. For example beads may be formed which comprise some aggregated colloid on the surface. It is possible to then pass such beads back through the bead making process, with adding any metal colloid. In this manner an additional polymer cover or layer is created which contains no metal colloid particles on the surface of the bead. Nevertheless, in certain instances it may be desirable to have metal on the surface of the bead and this may be achieved using the metal colloid or by metal coating or lithographically depositing metal on the bead surface. An advantage of beads also including metal on their surface is that SERRS sensing can also be carried out on the surface of the bead which is additionally internally labelled.

Preferred classes of monomers and specific monomers include, but are not limited to, the following classes and derivatives thereof: acrylic acid and derivatives (e.g., 2-bromoacrylic acid, acryloyl chloride, N-acryloyl tyrosine, N-acryoyl pyrrolidinone), acrylates (e.g., alkyl acrylates, allyl acrylates, hydroxypropyl acrylate), methacrylic acid and derivatives (e.g., itaconic acid, 2-(trifluoromethyl) propenoic acid), methacrylates (e.g., methyl methacrylate, hydroxyethyl methacrylate, 3-sulfopropyl methacrylate sodium salt), styrenes (e.g., (2, 3 and 4)-aminostyrene, Styrene-4-sulfonic acid, 3-nitrostyrene), vinyls (e.g., vinyl chloroformate, 4-vinylbenzoic acid, 4-vinylbenzaldehyde, vinyl imidazole, 4-vinylphenol, 4-vinylamine, acrolein), vinylpyridines (e.g., (2,3, and 4)-vinylpyridine, 3-butene 1,2-diol), boronic acids (e.g., 4-vinylboronic acid), sulfonic acids (e.g., 4-vinylsulfonic acid), metal chelators (e.g., styrene iminodiacetic acid), acrylamides and derivatives (e.g., N-methyl acrylamide), methacrylamides and derivatives (e.g., N,N-dimethyl acrylamide, N- (3-aminopropyl)methacrylamide), alkenes (e.g., 4-pentenoic acid, 3-chloro-1-phenyl-1-propene) (meth)acrylic acid anhydride and derivatives (e.g., methacrylic anhydride), silicon-containing monomers (e.g., (3-methacryloxypropyl) trimethoxy silane, tetramethyldisiloxane), polyenes (e.g., isoprene, 3-hydroxy-3,7, 1 1-trimethyl-1, 6,10-dodecatriene), azides (e.g., 4-azido-2,3,5,6-tetrafluorobenzoic acid), thiols (e.g., allyl mercaptan). Acrylate terminated or otherwise unsaturated urethanes, carbonates and epoxies can also be used in this present invention, as can silicon-based monomers. Crosslinking agents that lend rigidity to the subject polymeric compounds are known to those skilled in the art, and include, but are not limited to, di-, tri-, tetra- and penta-functional acrylates, methacrylates, acrylamides, vinyls, allyls, and styrenes.

Silver, gold or copper- colloid surfaces, especially silver, are particularly preferred for use in the present invention. However, metal alloys can also be used.

The surface may be a naked metal or may comprise a metal oxide layer on a metal surface. It may include an organic coating such as of citrate or of a suitable polymer, such as polylysine or polyphenol, to increase its sorptive capacity. It can include dyes which covalently link onto the polymer.

The colloid particles are preferably aggregated in a controlled manner so as to be of a uniform and desired size and shape and as stable as possible against self-aggregation. Processes for preparing such unaggregated colloids are already known. They involve, for instance, the reduction of a metal salt (eg. silver nitrate) with a reducing agent such as citrate, to form a stable microcrystalline suspension (see P.C. Lee & D. Meisel, J. Phys, Chem. (1982), 86, p.3.391*).* This "stock" suspension is then aggregated immediately prior to use. Suitable aggregating agents include acids (eg. HNO₃ or ascorbic acid), polyamines (eg. polylysine, spermine, spermidine, 1,4-diaminopiperazine, diethylenetriamine, aminoethyl) -1,3-propanediamine, triethylenetetramine and tetraethylenepentamine) and inorganic activating ions such as Cl⁻, I⁻, Na⁺ or Mg²⁺. To increase control over the process, all equipment used should be scrupulously clean, and reagents should be of a high grade.

The colloid particles can be of any size so long as they give rise to a SERRS effect - generally they will be about 4 - 50 nm in cross-section, preferably 25 - 36 nm, though this will depend on the type of metal.

The beads of the present invention are extremely small, typically less than 1µm in diameter. Preferably the beads are about 10nm to 1mm in cross-section, more preferably about 10nm to 1µm in cross section, but obviously not smaller than the dye coated colloid particles which they encapsulate.

The beads of the present invention may contain a number of SERRS active nanoparticles (comprising of aggregated metal colloid and adsorbed SERRS active dye or dyes) each of which may be identically labelled or differently labelled in order to increase the multiplexing capability. That is, a single bead may comprise of one or more SERRS active dyes. If more than one dye is incorporated within a bead, the SERRS signal obtained, will be a combination of the SERRS signals obtained from the various dyes used and by varying the dyes and/or relative levels of dyes within a bead, a "library" of beads may be formed, with discernable SERRS signals.

The action of the silver nanoparticles with the polymer in the formation of the beads is to reduce the size of the beads. This provides additional control over the bead chemistry and means that a large range of sizes can be formed. Thus, once the polymer is formed the SERRS bead arrangement is locked in position so that the state of aggregation chosen is preserved. Further, the polymer protects the silver nanoparticles from the environment.

The polymer used in these beads may further comprise functional groups (e.g. carboxylic acids, active esters of carboxylic acids, alcohols, amines, epoxides, chloromethyl styryl groups, vinyl groups, thiol groups, maleimide and succinimide) or may be further derivitised by a linking group or groups, such that target molecules may react and become bonded to the surface of the particles. The advantage of these groups is that they can act as points to covalently or non-covalently attach a molecule to provide a specific capture or analysis. The term linking group refers to any group which facilitates adherence between one or more target molecules and one or more SERRS active particles. The adherence may comprise any sort of bond, including but not limited to covalent, ionic, hydrogen bonding, van der Waals forces, or mechanical bonding.

In a further aspect, the present invention provides a method of preparing SERRS active beads comprising the steps of:
1) forming an aggregated metal colloid comprising a SERRS active dye absorbed thereto;
2) mixing the aggregated metal colloid with a monomer in a suitable solvent; and
3) effecting polymerisation of the monomer so as to form polymer beads encapsulating the aggregated metal colloid.

Alternatively beads may first be made without aggregated colloid comprising a SERRS active dye absorbed thereto. The beads may then be heated (e.g. to 80°C) in order to render them porous and capable of taking of said aggregated colloid comprising a SERRS active dye absorbed thereto. Cooling the beads traps the colloid particles within the bead. Dye/colloid can be removed by raising the temperature and steeping the beads in a suitable buffer.

The polymer beads may be further reacted so as to add functional or linking groups to an outer surface of the bead thereby allowing a molecule to be attached to the bead.

Examples of suitable target molecules include, nucleic acids, proteins such as antibodies, aldehydes, thiols, amines, explosives, drugs of abuse, therapeutic agents, metabolites and environmental pollutants. However, the beads, due to their extremely small size may also find application alone, e.g. as a security marker. The beads may therefore be applied to a surface by being contained within for example an aerosol spray, paint, ink or the like. The beads would generally be invisible to the naked eye, but would be loaded with a particular SERRS active dye or dye combination such that a specific SERRS signal could be detected. This may be particularly applicable to prevent/minimise counterfeiting.

Attaching suitable species such as DNA sequences to the polymer surface carries out the analysis/labelling procedure. In this way, the analysis process and the SERRS process are separate and both can be optimised.

The beads of the present invention could have broader applicability as a labelling technology. They could be used for example both for protein and antibody detection. In the case of proteins, the simplest example would be to attach one of a pair of proteins to the SERRS active surface and attach a fluorescent label to the other protein. When the pair complex, the bead would show both fluorescence and Raman scattering and when the pair separate, the Raman spectra would be detected only on the bead. A spectrometer exists which could be adapted to provide both fluorescence and Raman scattering simultaneously.

Moreover, the beads could be further adapted to for example, assist their separation when in use. The beads may be attached to a surface specifically by derivatising the surface of the beads with, for example, a DNA molecule which recognises a target and contains a biotin group, and could be brought down on a streptavidin surface. Alternatively/additionally, the beads could for example be magnetised by the incorporation of magnetic species within and/or on the surface of the beads weighted by the addition of materials which would allow the particles to be separated on the basis of their weight. The magnetic beads could then be brought down by using a small magnet to, for example, concentrate the particles in the beam of the spectrometer.

Suitable magnetic species may be in the form of magnetic nanoparticles which are incorporated into the bead. Such magnetic nanoparticles may include any ferromagnetic material in nanoparticle form including iron, magnetite, ferrites and compounds of other metals such as nickel and cobalt.

Incorporation of the magnetic nanoparticles may be achieved by combining a solution comprising said magnetic nanoparticles with a colloid/dye mixture and thereafter forming the SERRS active bead. The formed beads may thereafter be functionalised, as previously described.

In the case of antibodies, a very simple assay could be developed which eliminates some of the uncertainties with competitive and displacement assays in that, an antigen labelled with a fluorophore could be added to an antibody immobilised on the SERRS active bead. When the beads are removed from solution by centrifugation or magnetic fields, those beads with fluorophores can be interrogated and the Raman code read to determine which labels had been displaced. Alternatively, the beads could be used as specific labels in a sandwich assay in which the antigen is trapped on an antibody on a surface such as a plate or magnetic bead and A SERRS labelled antibody coated particle will adhere to the surface by attaching to the trapped antigen. The antigen may also initially be trapped by the antibody on the labelled particle. Protein recognition may be probed similarly by attaching a bead to each of the two proteins or by attaching a bead to one protein and another label such as a fluorophore to the other.

In yet another embodiment of the invention, molecular imprinting methods can be applied to impart specific molecular recognition properties to the polymer shell via templating approaches (see for example WO 00/41723). This generic approach can in principle be applied to impart affinity to the polymer shell for a range of analytes, including analytes of clinical, dietary, forensic and environmental relevance.

The method for obtaining the SERRS spectrum, may be conventional. However, the following might apply to the spectroscopic measurements:
Typically, the methods of the invention will be carried out using incident light from a laser, having a frequency in the visible spectrum ie. ^{∼}380nm - 780nm, particularly between 400nm - 650nm (the exact frequency chosen will generally depend on the dye used in each case - frequencies in the red area of the visible spectrum tend, on the whole, to give rise to better surface enhancement effects). However, it is possible to envisage situations in which other frequencies, for instance in the ultraviolet (ie. 200nm - 400nm) or the near-infrared ranges (700nm - 1100nm), might be used. Thus, SERRS detection may be conducted between about 200nm - 1100nm.

The selection and, if necessary, tuning of an appropriate light source, with an appropriate frequency and power, will be well within the capabilities of one of ordinary skill in the art, particularly on referring to the available SERRS literature. To achieve highly sensitive detection, using SERRS, a coherent light source is needed with a frequency at or close to the absorption maximum for the dye. If lower sensitivities are required, the light source need not be coherent or of high intensity and so lamps may be used in combination with a monochromator grating or prism to select an appropriate excitation frequency.

Several devices are suitable for collecting SERRS signals, including wavelength selective mirrors, holographic optical elements for scattered light detection and fibre-optic waveguides. The intensity of a SERRS signal can be measured for example using a charge coupled device (CCD), a silicon photodiode, or photomultiplier tubes arranged either singly or in series for cascade amplification of the signal. Photon counting electronics can be used for sensitive detection. The choice of detector will largely depend on the sensitivity of detection required to carry out a particular assay.

Note that the methods of the invention may involve either obtaining a full SERRS spectrum across a range of wavelengths, or selecting a peak and scanning only at the wavelength of that peak (ie. Raman "imaging"). It is also possible to detect all Raman scattering using only a filter to remove reflected light, Raleigh scattering etc and a detector such as photodiode.

Apparatus for obtaining and/or analysing a SERRS spectrum will almost certainly include some form of data processor such as a computer.

Raman signals consist of a series of discrete spectral lines of varying intensity. The frequencies and the relative intensities of the lines are specific to the SERRS active dye being detected and the Raman signal is therefore a "fingerprint" of the dye. If a SERRS analyser is being used selectively to detect a mixture of dyes then it will be necessary to detect the "fingerprint" spectrum for identification purposes.

Once the SERRS signal has been captured by an appropriate detector, its frequency and intensity data will typically be passed to a computer for analysis. Either the fingerprint Raman spectrum will be compared to reference spectra for identification of the detected Raman active compound or the signal intensity at the measured frequencies will be used to calculate the amount of Raman active compound detected.

In summary, the development of polymer particles containing SERRS active particles for multiple labelling of analytes is described. The colloid particles are dye labelled, pre-aggregated and locked into the polymer so that the SERRS effect is switched on permanently and the analytical event is separately carried out by, for example, covalently linking a molecule to be detected to the polymer surface. The main advantages are that the particles give very intense, stable SERRS. For simple, sensitive identification of a specific particle, there is a huge multiplex labelling capability in suspension which is unrivalled by any other technique and the SERRS active nanoaggregates are protected from the environment. In turn, this enables identification and quantitation of specific target analytes such as DNA sequences or peptides *in-situ* at ultra-low concentrations in a complex mixture.

In a further aspect the present invention provides a method of detecting a target molecule in a sample, the method comprising the steps of:
a) providing a SERRS active bead(s) according to the present invention wherein the bead has been generated so as to have a specifically identifiable SERRS signal and the surface of which has been modified so as to have attached thereto a moiety capable of binding said target molecule;
b) contacting said bead(s) with the sample so as to allow any of said target molecule in the sample to bind to said bead(s); and
c) detecting said target molecule by way of detecting the specifically identifiable SERRS signal obtained from a bead or beads being bound to said target molecule.

Typically the target molecule may be an antibody or antigen, in which case the SERRS active bead is modified so as to have a corresponding antigen or antibody respectively, attached to the surface of the bead. A conventional sandwich assay could be employed where a first antibody is attached to a surface, for binding to a specific protein which may be in a sample. The bead may be derivatised to include a second antibody which is also specific for said protein. If the protein is present in a sample it effectively becomes sandwiched between the two antibodies and this can be detected by SERRS being carried out on the bead attached to the second antibody. Other molecules which associate naturally may also be detected such as a receptor/ligand complex, with one of said complex immobilised on the bead surface.

In a preferred embodiment the method is used to detect a particular nucleic acid sequence in a sample. In this case, the bead may be modified so as to have a molecule capable of specifically hybridising to a nucleic acid molecule which comprises the particular nucleic acid sequence, attached to the surface of the bead. The molecule capable of specifically hybridising may be of a sufficient length to enable a simple hybridisation and subsequent detection to occur. Alternatively the molecule capable of specifically hybridising to the target nucleic acid molecule may be a relatively short molecule, say less than 50 nucleotides, e.g. less than 25 nucleotides in length, designed to act as a primer to allow hybridisation to said nucleic acid molecule and chain extension therefrom. In this manner the molecule attached to the surface of the bead will generally be attached via its 5' end so that the 3' end is capable of binding and initiating chain extension. Typically a further primer designed to bind to the target nucleic acid molecule, at a distance, e.g. 100 nucleotides to 2000 nucleotides, from the bead bound primer, may also be utilised. This can allow generation of a double stranded molecule, which incorporates the target nucleic acid sequence, using an appropriate DNA polymerase and necessary dNTPs (deoxynucleotide tri-phosphates), well known to those skilled in the art.

Indeed an amplification reaction e.g. PCR can be carried out in order to effectively enhance the amount of target nucleic acid to be detected. The further primer may also be modified by suitable means so as to comprise a moiety which is capable of binding to a particular surface. For example the primer may comprise a biotin molecule at the 5'-end of the primer so as to enable any amplified product to be capable of binding to a surface coated with streptavidin. In this manner an amplified product can be generated which can be bound to a surface via one end of the amplified product, whilst the free end incorporates a SERRS active bead which can easily be detected.

Due to the ability of being able to generate SERRS active beads with specific different SERRS signals it is possible to conduct what is known in the art as a multiplex reaction using a number of differently labelled beads each with different immobilised binding moieties e.g. oligonucleotides. In the case of oligonucleotides, each one may be designed to identify, for example, a particular polymorphism in a nucleic acid molecule, by incorporating specific sequences at the 3' end which will only allow chain extension to occur when complementary binding at the 3' end occurs. That is, when the nucleotide at the 3' end does not hybridise to the target, chain extension cannot occur therefrom. By carrying out an amplification reaction using another primer designed to generate an amplified product, it is possible to easily detect which polymorphism is present in a target nucleic acid molecule.

If an amplification reaction is carried out, a dye, such as Sybr Green can be used as an initial indicator that an amplified product has been obtained. This product can thereafter be identified by carrying out SERRS detection. This optional step can be employed such that only reactions which are identified as generating an amplified product are thereafter analysed by SERRS detection.

In summary the key advantages of the present invention are,
**1) Sensitivity:** Each bead is a separate sensing unit capable of housing up to millions of SERRS active dye labels. The entire available silver surface can be closely packed with dye labels. The intra molecular quenching mechanisms that prevent close proximity of two labels with, for example, fluorescence, do not apply in SERRS. Thus for one bead, the signal is very strong and easily and quickly detected at low concentration.
**2) Robustness:** The process of aggregation, essential to obtain the maximum signal from SERRS, is carried out during formation of the bead. Thus, it is preset and protected from the environment by the polymer making the signal long-lived, constant and unaffected by any biological event. In addition, the sensing species used to find a specific analyte is covalently attached to the polymer surface.
**3) Multiple detection:** The beads may be manufactured so as to contain many codes "written" by using different dye mixtures in each of a number of colloidal suspensions and by adding aggregated colloid and dye from more than one suspension to a specific bead. The sharp nature of the SERRS peaks then enables recognition of each bead type in a mixture of beads without any separation. Since all chromophores that adhere strongly to the SERRS active surfaces can be used, much simpler and more extensive dye chemistry is possible than with fluorescence. For example, the pattern of SERRS signals from a dye such as an azo is very sensitive to substitution round the chromophore making it much easier to create multiple labels than is the case with fluorescence. This greatly increases the coding potential with relatively simple dyes.

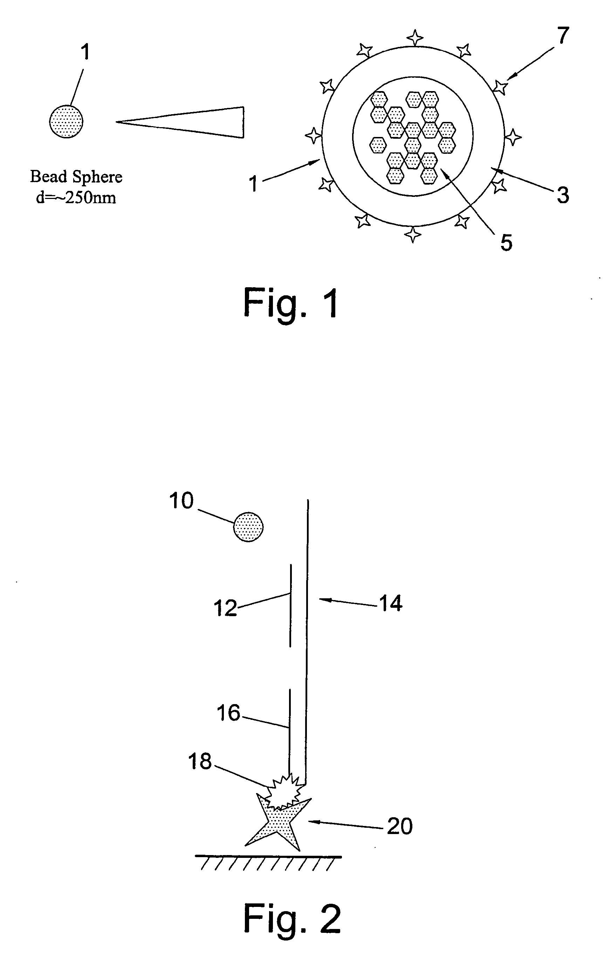

Moreover, because the beads include a metal, a significant mass is provided to the bead. This weight may also be used in detection and/or separation techniques employing the beads. For example, a DNA strip recognised as in Figure 2 could be trapped on the surface of a streptavidin coated plate of a quartz microbalance balance and the mass change detected. The heavy nature of the bead would make this mass change simple to detect increasing the sensitivity of the quartz balance compared to conventional trapping of the DNA strip. After detecting the weight change, the specific particle causing it could be detected by a weight change.

The present invention will now be further described by way of example and with reference to the Figures which show:
Figure 1 shows a schematic representation of a SERRS active bead according to the present invention;
Figure 2 shows in schematic a potential use of a SERRS active bead according to the present invention;
Figure 3 shows the Raman spectra of control beads and the SERRS of blank beads in Example 2;
Figure 4 shows the SERRS of a dye in a dye coated bead sample;
Figure 5 is a SEM image of dye coated beads, the measurement bar is 10 µm in length and the average diameter of the beads shown in ∼ 0.8 µm;
Figure 6 shows the SERRS spectra of ABT DMOPA, 8HQPK and a 1:10 ratio mixture of both dyes;
Figure 7 shows the SERRS spectra 8HQPK, benzotriazole rhodamine B and a 3:1 mixture of both dyes; and
Figure 8 shows a schematic representation of how a SERRS active bead according to the present invention may be used to identify a specific nucleic acid sequence, using an amplification reaction.

### Example section

### Example 1

### Colloid Preparation

Citrate-reduced colloid was prepared according to the method of Lee and Meisel (Rodger, C., Smith, W.E., Dent, G., Edmonson, M., J. Chem. Soc, Dalton Trans., 1996,5,716; Munro, C.H., Smith, W.E., Garner, M., Clarkson, J. and White, P.C., Langmuir, 1995, 11, 3712). All glassware was throughly cleaned using Aqua Regia (3:1, HCl:HNO₃) and then thoroughly rinsed with water followed by distilled water. Silver nitrate (90 mg) was dissolved in distilled water (500 ml) at a temperature of 40°C. The solution was then quickly heated using a Bunsen burner to a temperature of 98°C. At this point 10 ml of a 1% solution of tri-sodium citrate was added. The temperature was then carefully controlled, keeping the temperature constant at 98°C for 90 minutes. After this period of constant temperature the colloidal suspension was cooled and its UV absorbance spectrum measured. Colloid with a maximum UV absorbance within the range 403 - 410 nm was used in the experiments detailed below.

The silver pellets used in the polymerisations were prepared by centrifuging 12 tubes containing 12 ml of citrate-reduced colloid (λₘₐₓ of 406nm) at 3000 r.p.m. for 20 minutes. The supernatant from each tube was removed and the remaining silver was concentrated into one tube. The concentrated pellet was then re-suspended in acetonitrile using ultrasonication, which was then centrifuged at 3000 r.p.m. for 40 minutes. The supernatant was removed once again, the resultant pellet re-suspended in acetonitrile using ultrasonication (5 ml) and the suspension incorporated into the polymerisation.

The dye-loaded samples were prepared in exactly the same way as above, but in the 12 tubes containing citrate-reduced colloid 150 µl of a 1x10⁻⁶M solution of ABTDMOPA[4(5'-azobenzotriazoyl)-3,5-dimethoxyphenylamine] was added.

### Bead Synthesis

In what follows the *Control* polymer contained no colloid whereas the *Blank* polymer contained colloid but no dye. The Dye-Loaded polymer contained colloid and dye.

Three polymerisations (Control, Blank and Dye-Loaded) were carried out in three separate 50 ml thick-walled glass tubes. The reaction vessel for the Control polymer was charged with acetonitrile (25 ml), divinylbenzene 80 (0.5 g) and azobisisobutyronitrile (AIBN) (0.005 g), the reaction vessel for the Blank polymer was charged with acetonitrile (20 ml), the dye-free suspension of colloid in acetonitrile (5 ml), divinylbenzene 80 (0.5 g) and AIBN (0.005 g), and the reaction vessel for the Dye-Loaded polymer was charged with acetonitrile (20 ml), the dye-loaded suspension of colloid in acetonitrile (5m1), divinylbenzene 80 (0.5 g) and AIBN (0.005 g). The reaction vessels were shaken to dissolve the monomer and the AIBN, cooled on an ice-water bath for ^{∼}10 minutes and then sparged with oxygen-free nitrogen gas to remove dissolved oxygen. The tubes were then sealed and polymerisation effected by heating at 60°C on a low-profile roller for a period of 24 hours. Following polymerisation the polymer beads were isolated by filtration on membrane filter (0.2 µm), washed with several volumes of fresh acetonitrile and then dried *in vacuo* at 40°C.

### Bead Modification

1-Ethyl-3-(3-dimethylaminopropyl) carbodiimide (EDC) (500 mg) in 25 ml of 0.1 M MES buffer (pH 4.5) was added to the polymer beads and incubated at 25°C for two hours to form the amine surface active species. Figure 1 shows a schematic of a bead (1) prepared as described above. The bead (1) is formed of polymer shell (3) encapsulating aggregated colloid and SERRS active dye beads (5). The surface of the polymer shell (3) has been functionalised so as to have amino surface active species (7) which are capable of reacting with DNA. 3 µg of oligo per gram of bead in 25 ml of sodium phosphate buffer (pH 7.2) were added to the activated beads and reacted at 25°C overnight. The beads were removed from solution by centrifugation and re-suspended in phosphate buffer (pH 7.2) ready for use.

In a simple example (see Figure 2) of the use of the beads (1) prepared as described above, the oligonucleotide (12) is designed to recognise a specific sequence of DNA. When the target sequence (14) is present in a DNA extract, it will hybridise to the oligonucleotide (12) and thus be captured by the bead (10). A second oligonucleotide (16) containing for example a biotin group (18) is also hybridised and the system brought out on a streptavidin plate or bead (20). This assay specifically recognises the target sequence (14), which hybridised both the particle (10), and the biotinylated sequence (16). Alternatively, if magnetic particles are added to the bead, it and the hybridised target could be brought down on a suitable surface without the biotin step. In both methods various approaches can be used to remove the unincorporated probe. In the first method, no label is brought down if extra biotin is added to the streptavidin plate and consequently it is an interferent that does not need to be rigorously removed. However, the size of the complex created with the label makes separation easy and in either method removal of an unincorporated probe can be achieved using a flow cell system or a short chromatography step. Alternatively magnetic separation can be achieved by using magnetically coated streptavidin beads to pull the complex from solution.

### Example 2

A further set of silver pellets were then prepared using 12 centrifuge tubes. Blank beads contained 9.6 ml colloid and 2.4 ml water per tube. Dye coated beads contained 2.4 ml dye (1x10⁻⁶M) and 9.6 ml colloid per tube.

All tubes were left overnight after adding the dye and centrifuged for 40 minutes. Pellets were re-suspended in 10ml acetonitrile and re-centrifuged for a further 40 minutes (no ultrasonication)

Pellets were then re-suspended in a further 5 ml of acetonitrile and ultrasonicated for 2 minutes before addition to the polymer reaction mixture.

0.5g divinylbenzene and 0.005g AIBN were then dissolved in a total volume of 25 ml acetonitrile (including the 5 ml of colloid). Samples were degassed with N₂ for 10 minutes. The samples were then placed on rollers at 37°C, heated to 60°C and left to polymerise for 24 hours.

The samples were then filtered through a 0.2 mm pore size filter paper, washed with acetonitrile and dried in a vacuum oven.

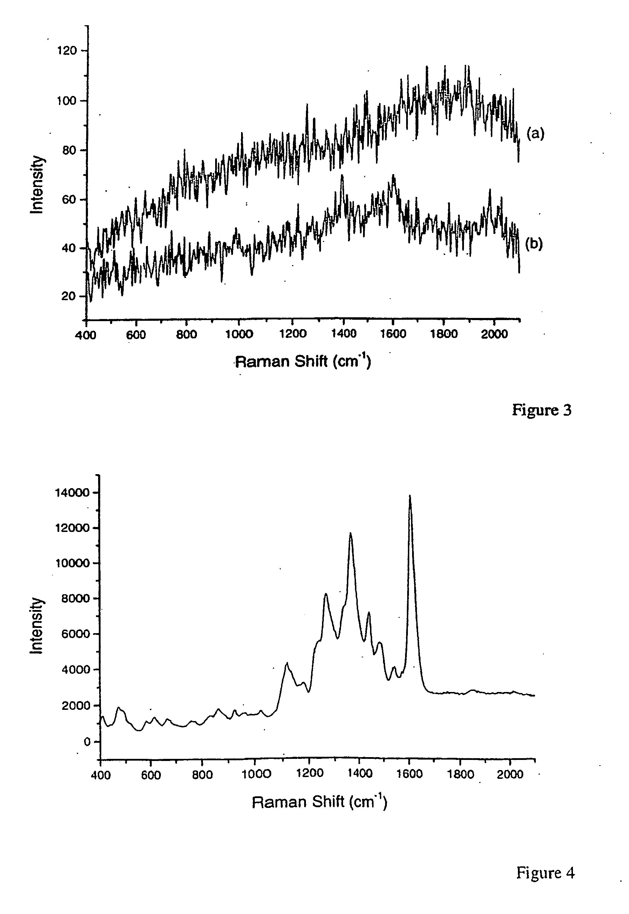

A small amount of the beads (i.e. control, blank and dye containing beads) were then suspended in ^{∼}1 ml of acetonitrile, ultrasonicated, dropped onto a microscope slide and allowed to dry. The spectra shown in Figures 3 and 4 were then recorded on the system 100 Renishaw microprobe Raman spectrometer, x20 objective, 100% power ^{∼}8mW) ls, la.

Figure 3 shows the Raman of control beads which are identified as (a) and the SERRS of blank beads which are identified as (b)

Figure 4 shows the SERRS of the dye in the dye containing bead sample.

### SEM images

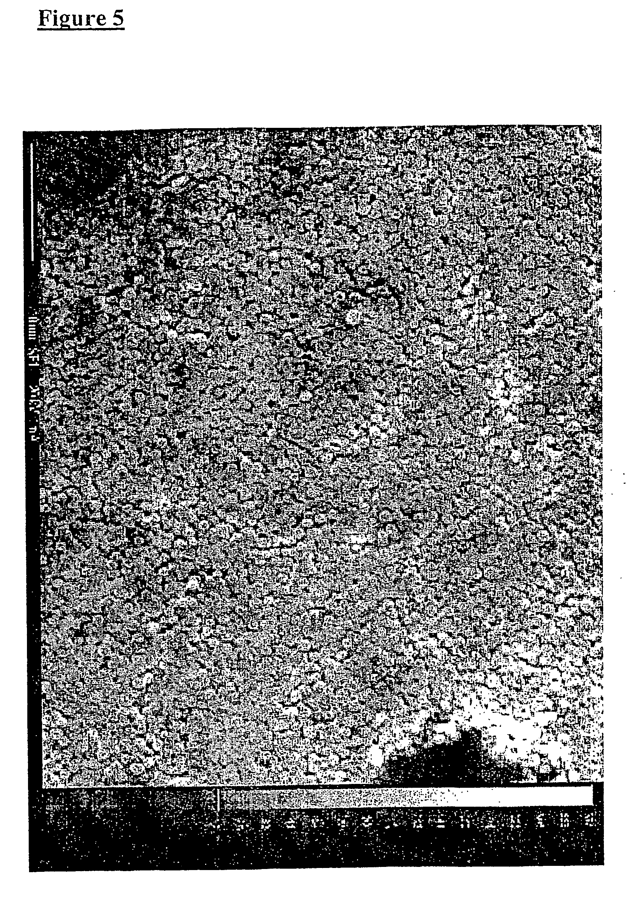

A small amount of the beads were then suspended in a solution of PVA using ultrasonication. The beads were then dried into a small glass disc, dried and stored under vaccum before analysis. The SEM image in Figure 5 was then recorded on the Cameca SX100 Electron Microprobe Microanalyser using 5 kV electron beam. The large bead is ^{∼}1.5 µm and the small beads are ^{∼}0.5 µm.

### Dye mixtures in PVA

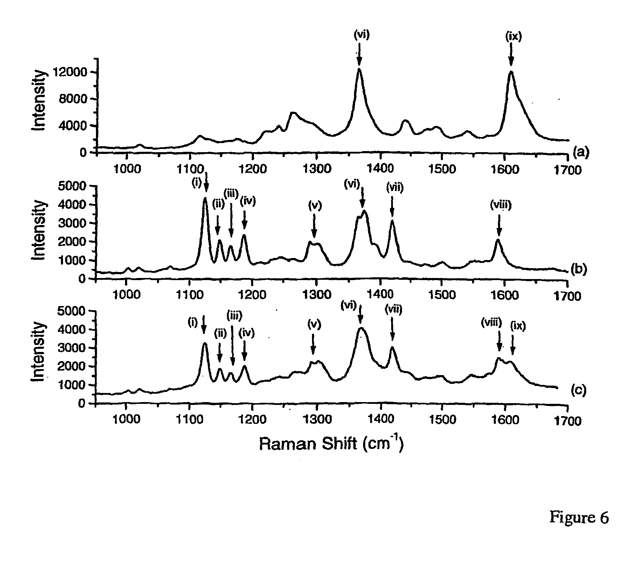

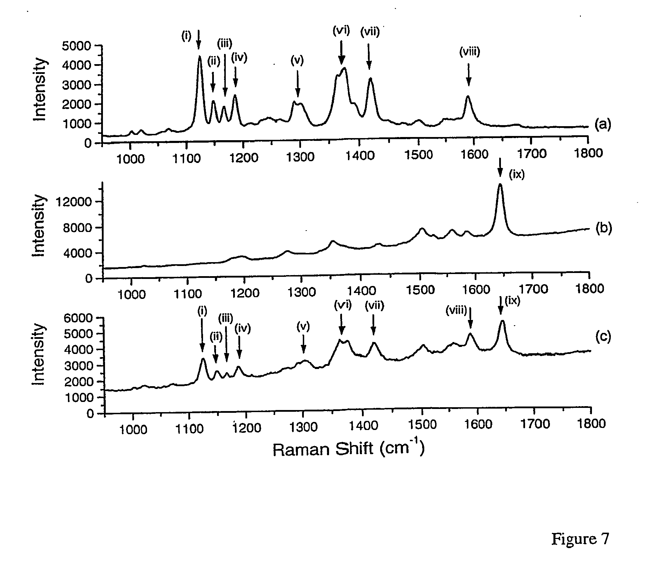

Figures 6 and 7 are Raman spectra of two examples of two mixtures in the PVA stable substrates.

Figure 6 shows the SERRS spectra of (a) ABT DMOPA (GMl9) (b) 8HQPK, and (C) a 1:10 ratio mixture of both dyes. The spectra were collected on the Renishaw microscope system, 514, 5s, la, 100% power (approx. 3mW), X5 objective.

In Figure 6, the main peaks of each of the two dyes (a) ABT DMOPA and (b) 8HQPK can be clearly identified in the spectrum of the mixture (c). Peaks labelled (i-v), (vii) and (viii) all originate from the 8HQPK dye (i.e. 1-[4-(8-hydroxy-quinolin-5ylazo)-phenyl]-ethanone) while the peak labelled (ix) is from the ABT DMOPA dye. The peak at ^{∼}1360cm⁻¹ in the mixture spectra (c) is a combination of the peaks at that position from both dyes. The intensity of this peak has increased relative to the other peaks in the spectrum.

Figure 7 shows the SERRS spectra of (a) 8HQPK, (b) Benzotriazole rhodamine B and (c) a 3:1 ratio mixture of both dyes. Collected on the Renishaw microscope system, 514nm, 5s,3a, 100% power (approx. 3mW), x5 objective. In Figure 7, the main peaks of each of the two dyes (a) 8HQPK and (b) BTRB can be clearly identified in the spectrum of the mixture (c). Peaks labelled (i-viii) all originate from the 8HQPK dye (i.e. Benzotriazole Rhodamine B - {9-[4-(Benzotriazol-5-yl sulfamoyl)-2-methyl-phenyl]-6-diethylamino-xanthen-3ylidene}diethylamonium) while the peak labelled (ix) is from the BTRB dye.

### Example 3 - Use of magnetic beads

### Bead Preparation

50ml of a solution of magnetic nanoparticles was added to 50ml of silver colloid treated with the dye mixture as in Example 1. The mixture of nanoparticles was then centrifuged and the bead process carried out as in Example 1. The bead was then functionalised as in Example 1 with oligonucleotides.

### Method (a)

This method combines separation and purification using the magnetic properties of the particle.

Using a sample of the PCR product intended for the assay in example 2 where a biotin strip and a bead have been hybridised to the target DNA, a small flat tip from an electro magnet was introduced into a micro titre plate well containing PCR product. The magnet was switched on and all magnetic beads collected. The magnet was then removed from the solution, dipped into a second well to wash it and then into a third well with a streptavidin coated area as in example 2. The electro magnet was switched off in the third well and the beads released. Following a period of two minutes in which the beads were allowed to attach to the streptavidin area, the magnet was switched on again. This removed all unattached beads.

In this way, all unincorporated biotin primer remained in the first well and all beads not attached to the target strip were removed in the first step and biotin labelled DNA not attached to the target strip was removed in the second step. The beads remaining in the foot of the well after the second step were interrogated as in Example 2 and the SERRS code used to identify the bead and hence the target DNA.

### Method (b)

This method illustrates the selectivity advantage of the use of derivatised beads containing only magnetic particles with no labelled silver particles. Commercial derivatised beads could also be used.

A batch of beads were prepared as described in Example 1 but without the addition of the silver nanoparticles and derivatised using specific oligonucleotide sequences to recognise a specific target DNA. Also incorporated into the PCR mix were other beads with the silver labels incorporated into the bead and no magnetic particles. They contain a second oligonucleotide sequence designed to recognise a different region of the same target DNA. Following PCR, the electromagnet is added to the well and switched on. Only samples with magnetic beads are attracted to its tip. The tip was then interrogated under the Raman microprobe. Only tips containing labelled beads were observed. This method is effective because it concentrates all the beads in the microscope interrogation volume. This increases sensitivity. It is reliable and selective because it involves a double hybridisation event.

### Method (c)

This method illustrates the combined use of Raman and fluorescence with magnetic beads.

Using the PCR mix from example 4 where Sybr green has been added to the DNA, the electromagnet was dipped into the PCR mix. It was switched on and the magnetic beads removed. The mix was then directly interrogated under the Raman microscope. A frequency was chosen so that the fluorescence and the Raman signals were detected on separate arts of the CCD detector. The method is effective because of the sensitivity created by collecting the beads in a small area for immediate detection. The fluorescence from Sybr green detects a hybridisation even within a conventional PCR machine so that a single well can be selected from an array for a fast one step identification analysis.

### Example 4 - Use of SERRS Beads for DNA Detection

In this example SERRS Beads can be used as a label for detection of a specific DNA sequence. The format is based around a PCR assay to demonstrate the high sensitivity of the SERRS Beads and also the increased capacity for multiplexing. An initial fluorescence screen can indicate the tubes or wells that have produced a response. These "hot" wells can be examined by SERRS and the actual DNA sequence or sequences that have hybridised determined.

Modified beads can be prepared according to Example 1 in which an outer surface of the bead has been modified so as to allow immobilisation of an oligonucleotide the 3' end of which is directed away from the outer surface of the bead. This can easily be achieved by for example attaching a group to the 5' end of the oligonucleotide during its synthesis and modifying the surface of the bead so that the group on the 5' end adheres to the surface of the bead.

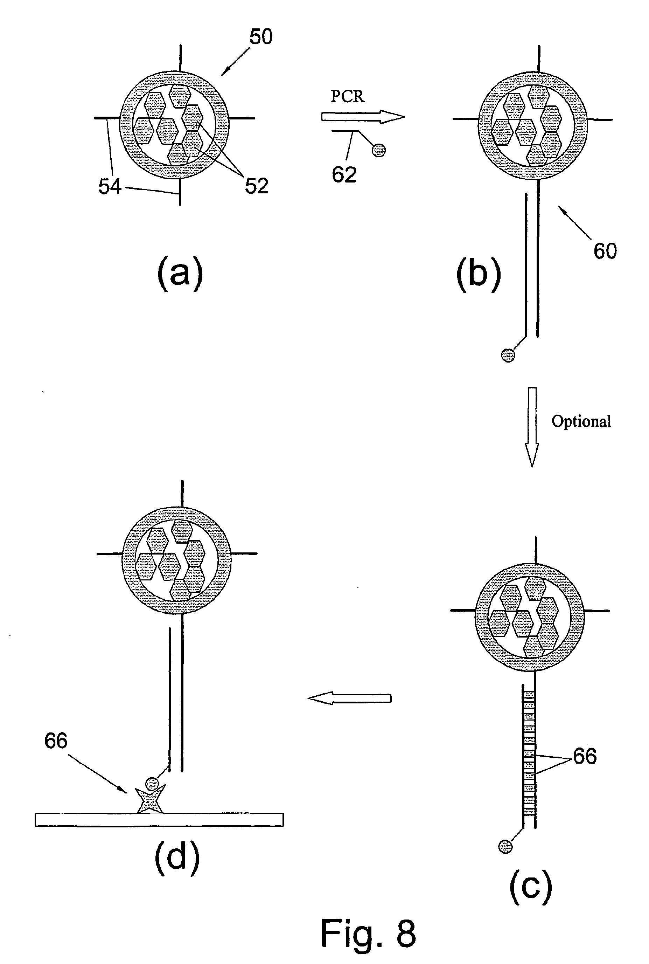

Figure 8a shows a schematic representation of such a bead 50, which has a specific SERRS signature by virtue of the dye labelled colloid particles 52 found within the bead 50, with a number of identical oligonucleotides 54 adhered to the surface of the bead 50.

### PCR Amplification of the Target

PCR is used to amplify a specific piece of double stranded DNA from the sample. This makes use of the existing specificity of PCR by using allele specific oligonucleotides for genotyping. Initially, primer sequences can be chosen to detect the mutational status of the most common mutation found in a particular disease. To analyse the genotype of the sample DNA, two forward primers may be used along with one reverse primer in a multiplexed amplification refractory mutation system, ARMS. The forward primers are immobilised via a 5'-terminus linker, onto different SERRS Beads that act as the labels for the specific primer. , The reverse primer is tagged at the 5'-terminus with biotin. As the PCR progresses the product will be built up on the SERRS Bead with biotin incorporated at the end of the amplicon away from the SERRS Bead. Alternatively, it is possible to carry out a conventional PCR of the target followed by introduction of the SERRS Beads when there is an issue with performing PCR on the SERRS Beads.

In the case of for example a pentaplex reaction five different primers can be immobilised on five different SERRS Beads and the PCR performed with all the beads mixed together with the sample.

Figure 8b shows schematically a labelled product 60 which has been obtained following a PCR reaction. Amplification using the primer attached to a SERRS bead (see Figure 8a) and a biotinylated primer 62 results in the generation of a biotinylated product which also incorporates a SERRS reactive bead.

### Initial Screening of the PCR [optional]

The success of the PCR can be measured by the increase in fluorescence of, for example, Sybr Green (64, see Figure 8c) as the amount of double stranded DNA increases. Use of a dissociation curve at the end of the PCR will confirm the presence of PCR product and not primer dimer. Thus if there is any amplification then the fluorescence will show this. It is possible to use a QPCR instrument to measure the increase in fluorescence during the PCR however a conventional plate reader could also be used after the PCR to rapidly analyse the tubes. Control tubes of no template controls and Beads with nonsense DNA can be used a deemed appropriate.

### Immobilisation of the SERRS Beads on a Surface

Once the tubes that have an increase in fluorescence had been optionally identified as having PCR product, the contents can be spotted onto a streptavidin coated slide (66, see Figure 8d). This immobilises the biotinylated PCR products with the SERRS Beads on the surface of the slide. The biotinylated primers will also be immobilised (but will not have a specifically detectable SERRS signal) but everything else can be washed away in a series of washes. (This will include the intercalated Sybr Green which could fluoresce and interfere with the SERRS signals). Thus, as series of SERRS Beads will now be immobilised on the surface of the slide ready for interrogation by SERRS.

### SERRS Analysis of the Immobilised Beads

SERRS spectra can be taken on the immobilised SERRS Beads to identify the actual primer sequences that have been used to generate the PCR products. The ability of SERRS to identify the components of a mixture without separation can be used to identify the SERRS Beads from particular tubes with a multiplex e.g. pentaplex likely to be possible by eye although statistics can also be used to confirm this for the non-specialist with a view to increasing the degree of multiplexing.

Typically the whole setup should take little more than the time for a standard QPCR experiment i.e. about 150 mins if a dissociation curve is used or about 60 mins if less cycles are used in a standard PCR cycler. The PCR setup can be arranged in such a way that the effective concentration of the primers on the beads and the final volume will be entered into a spread sheet and all other values automatically displayed. This makes setting up the PCR very simple and reduced systematic error in the calculations. After the PCR has finished there may be a need to pipette the contents of the strip tubes into microtitre plates for either plate reading or to be compatible with robotic handling. Alternatively direct pipetting onto the streptavidin slide is an option.

It may be possible to use linear extension as opposed to amplification to incorporate the SERRS Bead into a long double stranded piece of DNA. Fluorescence detection is unlikely to pick this up but the SERRS Beads will report on the enzymatic extension whether there is one piece of DNA or millions.

### Example 5 - SERRS bead DNA binding assay, exploiting the biotin - streptavidin binding affinity

An example of the SERRS beads binding assay is presented here. In this example the 110 base long sequence of a mutant Cystic Fibrosis gene is analysed.

The active surface of a SERRS bead, labelled with an azo dye, was derivatised with a short oligonucleotide probe, complementary to a sequence of DNA found on a mutant Cystic fibrosis gene. The mutant CF strand was linked to a biotin group at the opposite end of the sequence to where the complementary probe was synthesised. The SERRS-DNA probe was hybridised to the CF-biotin sequence and immobilised on a streptavidin plate.

### Method

The SERRS beads were synthesised using the method outlined earlier with divinyl benzene 80 and methacrylic acid. The surface of the beads is covered with a number of carboxylic acid groups. These active acid groups are used for attaching oligonucleotides and other molecules to the surface of the bead.

### DNA sequences

Two sequences were synthesised using the Expedite DNA Synthesiser
Sequence A containing an amine linker, X.
Sequence A -X-GTATCTATATTCATCATAGGAAACACCATT
Sequence B containing biotin, B
Sequence B -B-GACTTCACTTCTAATGATGATTATGGGAGA
(X = aminohexamethylene and is added to the 5'-terminus to produce an oligonucleotide with a primary amine group at the end of a carbon chain after solid phase synthesis and purification).

Sequence A (400µl, 0.6mg/ml) was purified and coupled to SERRS active beads (70mg) using 1-ethyl-3-(3-dimethylaminopropyl) carbodiimide (EDC, 37mg) and N-hydroxysuccinimide (24mg) in 0.1M PBS (750µl) buffer and acetonitrile (100µl).

Sequence B (0.5µM) was used in the PCR amplification reaction with its CF complement and a reverse primer. The product was purified and added to the oligo derivatised beads with additional Sequence B and heated to 95°C for 10mins.

After this 100µl of the dispersed solution was dotted onto a streptavidin plate and left to stand for 3hrs in a humid chamber. The DNA derivatised beads were used as a control. The plate was washed with 1% SDS solution, water and acetonitrile. The beads were dried and examined by Raman photospectroscopy.

## Claims

1. A bead for use in identification of a target molecule using SERRS, wherein the bead comprises a plurality of nanoparticles, said nanoparticles comprising aggregated metal colloid and a SERRS active dye or dyes absorbed thereto, wherein each of said nanoparticles provides an identical SERRS signal, or are provided with differing dyes or dye combinations so as to provide distinguishable SERRS signals, encapsulated within a polymer shell, or shells.

2. A bead according to claim 1 wherein the polymer shell or shells is/are formed from a wide variety of monomers and cross linkers including monomers that polymerise via chain-growth mechanisms and step-growth mechanisms capable of forming a bead which encapsulates the aggregated colloid and SERRS active dye.

3. A bead according to any of claims 1 or 2 wherein said polymer shell or shells is/are formed from any of the following classes and derivatives thereof: acrylic acid and derivatives including 2-bromoacrylic acid, acryloyl chloride, N-acryloyl tyrosine, N-acryoyl pyrrolidinone, acrylatesincluding, alkyl acrylates, allyl acrylates, hydroxyypropyl acrylate, methacrylic acid and derivatives including itaconic acid, 2-(trifluoromethyl) propenoic acid, methacrylates including methyl methacrylate, hydroxyethyl methacrylate, 3-sulfopropyl methacrylate sodium salt, styrenes including (2,3 and 4)-aminostyrene, styrene-4-sulfonic acid, 3-nitrostyrene, vinyls including vinyl chloroformate, 4-vinylbenzoic acid, 4-vinylbenzaldehyde, vinly imidazole, 4-vinylphenol, 4-vinylamine, acrolein, vinylpyridines including (2,3 and 4)-vinylpyridine, 3-butene 1,2-diol, boronic acids including 4-vinylboronic acid, sulfonic acids including 4-vinylsulfonic acid, metal chelators (including styrene iminodiacetic acid, acrylamides and derivatives including N-methyl acrylamide), methacrylamides and derivatives including N,N-dimethyl acrylamide, N-(3-aminopropyl)methacrylamide, alkenes 4-pentenoic acid, 3-chloro-1-phenyl-1-propene (meth)acrylic acid anhydride and derivates methacrylic anhydride), silicon-containing monomers including (3-methacryloxypropyl)trimethoxy silane, tetramethyldisiloxane, polyenes isoprene, 3-hydroxy-3,7,1 1-trimethyl-1,6,10-dodecatriene, azides including 4-azido-2,3,5,6-tetrafluorobenzoic acid, thiols including allyl mercaptan.

4. A bead according to either of claims 1 or 2 wherein said polymer shell or shells is/are formed from acrylate terminated or otherwise unsaturated urethanes, carbonates and epoxies or silicon-based monomers.

5. A bead according to any preceding claim wherein said polymer shell or shells comprises crosslinking agents that lend rigidity to the subject polymeric compounds such as di-, tri-, tetra- and penta-functional acrylates, methacrylates, acrylamides, vinyls, allyls, and styrenes.

6. A bead according to any preceding claim wherein the aggregated metal colloid comprises silver, gold, copper or metal alloy colloid surfaces.

7. A bead according to any preceding claim wherein the aggregated metal colloid comprises a naked metal or a metal oxide layer on a metal surface.

8. A bead according to any preceding claim wherein the aggregated metal colloid comprises an organic coating of citrate or of a polylysine or polyphenol polymer, to increase its sorptive capacity.

9. A bead according to any preceding claim wherein the aggregated metal colloid is in the form of aggregated metal colloid particle or particles.

10. A bead according to claim 9 wherein the aggregated metal colloid particles have a cross section of 4 - 50 nm, or 25 - 36 nm.

11. A bead according to any preceding claim wherein the bead has a cross-section of less than 1 µm.

12. A bead according to any preceding claim wherein the outermost polymer shell further comprises functional groups selected from any of the following: carboxylic acids, active esters of carboxylic acids, alcohols, amines, epoxides, chloromethyl styryl groups, vinly groups, thiol groups, maleimide and succinimide, or is further derivitised by a linking group or groups, such that target molecules can react and become bounded to the surface of the bead, or such that a moiety may react and become bounded to the surface of the beadwhich moiety is capable of binding said target molecule.

13. Use of a bead according to any preceding claim in labelling technology for protein and antibody detection.

14. Use of a bead according to any preceding claim for
molecular imprinting methods, wherein specific molecular recognition properties, are imparted to the polymer shell via templating approaches.

15. A bead according to any preceding claim further comprising a magnetic particle or particles encapsulated within the polymer bead, for rendering the SERRS active bead magnetic.

16. A library of beads according to any preceding claim wherein each bead comprises a discernable SERRS signal, the discernable SERRS signal being obtained by varying the SERRS active dyes and/or relative levels of the dyes within each bead.

17. A method of preparing beads according to any preceding claims comprising the steps of:
a) forming nanoparticles of an aggregated metal colloid comprising a SERRS active dye absorbed thereto;
b) mixing the aggregated metal colloid with a monomer in a suitable solvent; and
c) effecting polymerisation of the monomer so as to form polymer beads encapsulating the nanoparticles of dye absorbed aggregated metal colloid.

18. A method of preparing beads according to any preceding claims comprising the steps of:
a) providing a monomer in a suitable solvent;
b) effecting polymerisation of the monomer so as to form polymer beads;
c) adding nanoparticles of aggregated metal colloid comprising a SERRS active dye absorbed thereo to a solution comprising the polymer beads; and
d) heating the solution so as to cause the nanoparticles of dye absorbed aggregated metal colloid to be taken up and become encapsulated by the polymer beads.

19. A method according to claims 17 or 18 wherein the polymer beads are further reacted so as to add functional or linking groups to an outer surface of the bead thereby allowing a molecule to be attached to the bead.

20. Use of a bead or beads according to any of claims 1-15 in a method of detecting a target molecule in a sample, the method comprising the steps of:
a) providing a bead or beads according to any one of claims 1 - 15 wherein the bead has been generated so as to have a specifically identifiable SERRS signal and the surface of which has been modified so as to have attached thereto a moiety capable of binding said target molecule;
b) contacting said bead or beads with the sample so as to allow any of said target molecule in the sample to bind to said bead or beads; and
c) detecting said target molecule by way of detecting the specifically identifiable SERRS signal obtained from a bead or beads being bound to said target molecule.

21. The use according to claim 20 wherein the target molecule is an antibody, antigen, receptor, ligand or nucleic acid molecule, such as a DNA molecule.

22. The use according to claim 21 wherein the moiety capable of binding said target molecule is an antigen, antibody, ligand, receptor or complementary nucleic acid respectively.

23. The use according to any one of claims 20 - 22 wherein the bead or beads are magnetic such that the bead or beads may easily be separated and/or concentrated after step b).

24. Use of a bead or beads according to any one of claims 1-15 in a method of detecting a specific nucleic acid in a sample, comprising the steps of:
a) providing a bead or beads according to any one of claims 1 - 15 wherein the bead has been generated so as to have a specifically identifiable SERRS signal and the surface of which has been modified so as to have attached thereto a moiety capable of binding said target molecule wherein the moiety is an oligonucleotide capable of specifically hybridising with a target nucleic acid;
b) contacting said bead or beads with the sample so as to allow any of said target nucleic acid in the sample to bind to said bead or beads;
c) contacting said target bound beads with a further labelled nucleic acid and said further labelled nucleic acid to specifically hybridise thereto;
d) optionally carrying out an amplification reaction and further optionally detecting if said amplification reaction has been successful;
e) contacting said labelled hybridised nucleic acid bond beads with a surface capable of binding said labelled moiety; and
f) detecting said target molecule by way of detecting the specifically identifiable SERRS signal obtained from said beads which have been effectively bound to said substrate.

25. The use according to claim 24 further comprising one or more washing steps to remove unbound reagents from steps b, c and/or e to be removed.

26. Use of a bead or beads according to any one of claims 1-15 in a method of detecting a target molecule in a sample, the method comprising the steps of:
a) providing a SERRS active bead or beads according to any one of claims 1 - 15 wherein the bead has been generated so as to have a specifically identifiable SERRS signal and a surface of which has been modified so as to have attached thereto a moiety capable of binding said target molecule;
b) providing a magnetic bead having attached thereto a moiety capable of binding said target molecule;
c) contacting said bead or beads and magnetic bead or beads with the sample so as to allow any of said target molecule in the sample to bind to said bead or beads; and
d) detecting said target molecule by way of detecting the specifically identifiable SERRS signal obtained from a bead or beads being bound to said target molecule.

27. The use according to claim 26, wherein step c) further comprises the step of removing the bead or beads from solution by centrifugation or magnetic field or fields.

28. Use of a SERRS reactive bead according to any one of claims 1 - 15 as a label in a sandwich assay.

29. The use according to claim 28 wherein an antigen to be detected is trapped by an antibody immobolised on a surface and an antibody coated on the SERRS reactive bead.

30. The use according to claim 29, wherein the surface is on a magnetic bead.

31. An antigen trapped by a bead according to any of claims 1 - 5 together with a trapped antigen; and a magnetic bead.

## Patentansprüche

1. Perle zur Verwendung zum Identifizieren eines Zielmoleküls unter Anwendung von SERRS, wobei die Perle eine Mehrzahl von Nanoteilchen umfasst, wobei die Nanoteilchen aggregiertes Metallkolloid und einen daran absorbierten, SERRS-aktiven Farbstoff oder Farbstoffe umfassen, wobei jedes der Nanoteilchen ein identisches SERRS-Signal bereitstellt, oder mit verschiedenen Farbstoffen oder Farbstoffkombinationen ausgestattet sind, um unterscheidbare SERRS-Signale bereitzustellen, und innerhalb einer Polymerschale oder -schalen eingekapselt sind.

2. Perle nach Anspruch 1, wobei die Polymerschale oder -schalen aus einer umfangreichen Reihe verschiedener Monomere und Vernetzungsmittel, einschließlich Monomeren gebildet ist/sind, die sich über Kettenwachstumsmechanismen und Schrittwachstumsmechanismen polymerisieren und in der Lage sind, eine Perle zu bilden, die das aggregierte Kolloid und SERRS-aktiven Farbstoff einkapselt.

3. Perle nach einem der Ansprüche 1 oder 2, wobei die Polymerschale oder -schalen aus einer der folgenden Klassen und Derivate davon gebildet ist/sind: Acrylsäure und Derivaten, einschließlich 2-Bromacrylsäure, Acryloylchlorid, N-Acryloyltyrosin, N-Acryloylpyrrolidinon, Acrylaten, einschließlich Alkylacrylaten, Allylacrylaten, Hydroxypropylacrylat, Methacrylsäure und Derivaten, einschließlich Itaconsäure, 2-(Trifluormethyl)propensäure, Methacrylaten, einschließlich Methylmethacrylat, Hydroxyethylmethacrylat, 3-Sulfopropylmethacrylat-Natriumsalz, Styrolen, einschließlich (2-, 3- und 4-)Aminostyrol, Styrol-4-sulfonsäure, 3-Nitrostyrol, Vinylen, einschließlich Vinylchlorformiat, 4-Vinylbenzoesäure, 4-Vinylbenzaldehyd, Vinylimidazol, 4-Vinylphenol, 4-Vinylamin, Acrolein, Vinylpyridinen, einschließlich (2-, 3- und 4-)Vinylpyridin, 3-Buten-1,2-diol, Boronsäuren, einschließlich 4-Vinylboronsäure, Sulfonsäuren, einschließlich 4-Vinylsulfonsäure, Metallchelatbildnern (einschließlich Stryroliminodiessigsäure, Acrylamiden und Derivaten, einschließlich N-Methylacrylamid), Methacrylamiden und Derivaten, einschließlich N,N-Dimethylacrylamid, N-(3-Aminopropyl)methacrylamid, Alkenen, -4-pentensäure, 3-Chlor-1-phenyl-1-propen(meth)acrylsäureanhydrid, und Methacrylsäureanhydridderivaten), siliciumhaltigen Monomeren, einschließlich (3-Methacryloxypropyl)trimethoxysilan, Tetramethyldisiloxan, Polyenen Isopren, 3-Hydroxy-3,7,1,1-trimethyl-1,6,10-dodecatrien, Aziden, einschließlich 4-Azido-2,3,5,6-tetrafluorbenzoesäure, Thiolen, einschließlich Allylmercaptan.

4. Perle nach einem der Ansprüche 1 oder 2, wobei die Polymerschale oder -schalen aus acrylatterminierten oder ansonsten ungesättigten Urethanen, Carbonaten und Epoxiden oder Monomeren auf Siliciumbasis gebildet wird/werden.

5. Perle nach einem der vorhergehenden Ansprüche, wobei die Polymerschale oder - schalen Vernetzungsmittel umfasst/umfassen, die den betreffenden polymeren Verbindungen, wie di-, tri-, tetra- und pentafunktionellen Acrylaten, Methacrylaten, Acrylamiden, Vinylen, Allylen und Styrolen Steife verleihen.

6. Perle nach einem der vorhergehenden Ansprüche, wobei das aggregierte Metallkolloid Silber-, Gold-, Kupfer- oder Metalllegierungskolloidoberflächen umfasst.

7. Perle nach einem der vorhergehenden Ansprüche, wobei das aggregierte Metallkolloid eine nackte Metall- oder eine Metalloxidschicht auf einer Metalloberfläche umfasst.

8. Perle nach einem der vorhergehenden Ansprüche, wobei das aggregierte Metallkolloid eine organische Beschichtung aus Citrat oder aus einem Polylysin oder Polyphenolpolymer zum Erhöhen seiner Sorptionsfähigkeit umfasst.

9. Perle nach einem der vorhergehenden Ansprüche, wobei das aggregierte Metallkolloid in Form von aggregiertem Metallkolloidteilchen oder mehreren Teilchen vorliegt.

10. Perle nach Anspruch 9, wobei die aggregierten Metallkolloidteilchen einen Querschnitt von 4 - 50 nm oder 25 - 36 nm aufweisen.

11. Perle nach einem der vorhergehenden Ansprüche, wobei die Perle einen Querschnitt von weniger als 1 µm aufweist.

12. Perle nach einem der vorhergehenden Ansprüche, wobei die äußerste Polymerschale des Weiteren funktionelle Gruppen umfasst, die von einem der Folgenden ausgewählt sind: Carbonsäuren, aktiven Estern von Carbonsäuren, Alkoholen, Aminen, Epoxiden, Chlormethylstyrylgruppen, Vinylgruppen, Thiolgruppen, Maleimid und Succimid oder noch weiter derivatisiert wird durch eine Verknüpfungsgruppe oder - gruppen, derart, dass Zielmoleküle mit der Oberfläche der Perle reagieren und daran gebunden werden können oder derart, dass ein Anteil mit der Oberfläche der Perle reagieren und daran gebunden werden kann, welcher Anteil in der Lage ist, das Zielmolekül zu binden.

13. Verwendung einer Perle nach einem der vorhergehenden Ansprüche in der Labeltechnologie zur Protein- und Antikörpererfassung.

14. Verwendung einer Perle nach einem der vorhergehenden Ansprüche für Molekulardruckverfahren, wobei spezifische molekulare Erkennungseigenschaften der Polymerschale durch Schablonieransätze verliehen werden.

15. Perle nach einem der vorhergehenden Ansprüche, des Weiteren ein oder mehrere magnetische(s) Teilchen umfassend, das/die innerhalb der Polymerperle eingekapselt ist/sind, zum Magnetischmachen der SERRS-aktiven Perle.

16. Bibliothek von Perlen nach einem der vorhergehenden Ansprüche, wobei jede Perle ein wahrnehmbares SERRS-Signal umfasst, wobei das wahrnehmbare SERRS-Signal durch Variieren der SERRS-aktiven Farbstoffe und/oder relativen Niveaus der Farbstoffe innerhalb jeder Perle erhalten wird.

17. Verfahren zur Herstellung von Perlen nach einem der vorhergehenden Ansprüche, umfassend die Schritte des:
a) Bildens von Nanoteilchen eines aggregierten Metallkolloids umfassend einen daran absorbierten SERRS-aktiven Farbstoff;
b) Mischens des aggregierten Metallkolloids mit einem Monomer in einem geeigneten Lösungsmittel; und
c) Durchführens der Polymerisation des Monomers, um Polymerperlen zu bilden, die die Nanoteilchen des farbstoffabsorbierten aggregierten Metallkolloids einkapseln.

18. Verfahren zum Herstellen von Perlen nach einem der vorhergehenden Ansprüche, umfassend die Schritte des:
a) Bereitstellens eines Monomers in einem geeigneten Lösungsmittel;
b) Ausführung der Polymerisation des Monomers, um Polymerperlen zu bilden;
c) Hinzugebens von Nanoteilchen von aggregiertem Metallkolloid umfassend einen daran absorbierten SERRS-aktiven Farbstoff zu einer Lösung, die Polymerperlen umfasst; und
d) Erhitzens der Lösung, um die Nanoteilchen von farbstoffabsorbiertem aggregiertem Metallkolloid dazu zu bringen, aufgenommen und durch die Polymerperlen eingekapselt zu werden.

19. Verfahren nach den Ansprüchen 17 oder 18, wobei die Polymerperlen noch weiter reagiert werden, um funktionelle oder Verknüpfungsgruppen an eine Außenfläche der Perle anzubringen und es einem Molekül dadurch zu gestatten, an der Perle befestigt zu werden.

20. Verwendung einer Perle oder von Perlen nach einem der Ansprüche 1 - 15 bei einem Verfahren zum Erfassen eines Zielmoleküls in einer Probe, wobei das Verfahren die Schritte umfasst des:
a) Bereitstellens einer Perle oder von Perlen nach einem der Ansprüche 1 - 15, wobei die Perle so erzeugt worden ist, dass sie ein spezifisch identifizierbares SERRS-Signal aufweist und deren Oberfläche so modifiziert worden ist, dass ein Anteil daran befestigt ist, der in der Lage ist, das Zielmolekül zu binden;
b) Kontaktierens der Perle oder Perlen mit der Probe, um zu gestatten, dass irgendeines der Zielmoleküle in der Probe sich an die Perle oder Perlen bindet; und
c) Erfassens des Zielmoleküls durch Erfassen des spezifisch identifizierbaren SERRS-Signals, das von einer Perle oder Perlen erhalten wird, die an das Zielmolekül gebunden ist/sind.

21. Verwendung nach Anspruch 20, wobei das Zielmolekül ein Antikörper, Antigen, Rezeptor, Ligand oder Nucleinsäuremolekül, wie beispielsweise ein DNA-Molekül, ist.

22. Verwendung nach Anspruch 21, wobei der Anteil, der des Bindens des Zielmoleküls fähig ist, jeweils ein Antigen, Antikörper, Ligand, Rezeptor oder eine komplementäre Nucleinsäure ist.

23. Verwendung nach einem der Ansprüche 20 - 22, wobei die Perle oder Perlen magnetisch sind, derart, dass die Perle oder Perlen ohne weiteres getrennt und/oder nach Schritt b) konzentriert werden kann/können.

24. Verwendung einer Perle oder von Perlen nach einem der Ansprüche 1 - 15, bei einem Verfahren des Erfassens einer spezifischen Nucleinsäure in einer Probe, umfassend die Schritte des:
a) Bereitstellens einer Perle oder von Perlen nach einem der Ansprüche 1 - 15, wobei die Perlen so erzeugt worden ist/sind, dass sie ein spezifisch identifizierbares SERRS-Signal aufweist/aufweisen und die Oberfläche derselben so modifiziert worden ist, dass ein Anteil daran befestigt ist, der in der Lage ist, das Zielmolekül zu binden, wobei der Anteil ein Oligonucleotid ist, das in der Lage ist, spezifisch mit einer Zielnucleinsäure zu hybridisieren;
b) Kontaktierens der Perle oder Perlen mit der Probe, um zu gestatten, dass irgendeine der Zielnukleinsäuren in der Probe sich an die Perle oder Perlen bindet;
c) Kontaktierens der zielgebundenen Perlen mit einer weiteren gelabelten Nucleinsäure, wobei die weitere gelabelte Nucleinsäure spezifisch daran hybridisiert;
d) wahlweisen Durchführens einer Amplifizierungsreaktion und des Weiteren wahlweise Erfassens, ob die Amplifizierungsreaktion erfolgreich gewesen ist;
e) Kontaktierens der gelabelten hybridisierten Nucleinsäure-gebundenen Perlen mit einer Oberfläche, die des Bindens des gelabelten Anteils fähig ist; und
f) Erfassens des Zielmoleküls durch Erfassen des spezifisch identifizierbaren SERRS-Signals, das von den Perlen erhalten wird, die effektiv an das Substrat gebunden worden sind.

25. Verwendung nach Anspruch 24, des Weiteren einen oder mehrere Waschschritte umfassend zum Entfernen ungebundener Reaktionsmittel aus den Schritten b, c, und/oder e, die entfernt werden sollen.

26. Verwendung einer Perle oder von Perlen nach einem der Ansprüche 1 - 15 bei einem Verfahren zum Erfassen eines Zielmoleküls in einer Probe, wobei das Verfahren die Schritte umfasst des:
a) Bereitstellens einer SERRS-aktiven Perle oder Perlen nach einem der Ansprüche 1 - 15, wobei die Perle so erzeugt worden ist, dass sie ein spezifisch identifizierbares SERRS-Signal aufweist und eine Oberfläche derselben so modifiziert worden ist, dass ein Anteil daran befestigt ist, der in der Lage ist, das Zielmolekül zu binden;
b) Bereitstellens einer magnetischen Perle, an die ein Anteil befestigt ist, der des Bindens des Zielmoleküls fähig ist;
c) Kontaktierens der Perle oder Perlen und magnetischen Perle oder Perlen mit der Probe, um zu gestatten, dass irgendeines der Zielmoleküle in der Probe sich an die Perle oder Perlen bindet; und
d) Erfassens des Zielmoleküls durch Erfassen des spezifisch identifizierbaren SERRS-Signals, das von einer Perle oder Perlen erhalten wird, die an das Zielmolekül gebunden wird/werden.

27. Verwendung nach Anspruch 26, wobei der Schritt c) des Weiteren den Schritt des Entfernens der Perle oder Perlen aus Lösung durch Zentrifugieren oder magnetisches Feld oder Felder umfasst.

28. Verwendung einer SERRS-aktiven Perle nach einem der Ansprüche 1 - 15 als Label in einem Sandwich-Assay.

29. Verwendung nach Anspruch 28, wobei ein zu erfassendes Antigen durch einen Antikörper, der auf einer Oberfläche immobilisiert ist, und einen schichtförmig auf die SERRS-reaktive Perle aufgebrachten Antikörper aufgefangen wird.

30. Verwendung nach Anspruch 29, wobei die Oberfläche sich auf einer magnetischen Perle befindet.

31. Antigen, das durch eine Perle nach einem der Ansprüche 1 - 5 zusammen mit einem aufgefangenen Antigen aufgefangen ist; und magnetische Perle.

## Revendications

1. Bille pour l'utilisation dans l'identification d'une molécule cible en utilisant le procédé SERRS, dans lequel la bille comprend une pluralité de nanoparticules, lesdites nanoparticules comprenant un colloïde métallique agrégé et un colorant ou des colorants actif(s) pour SERRS en absorption dessus, dans laquelle chacune desdites nanoparticules fournit un signal SERRS identique, ou sont proposées avec des colorants différents ou des combinaisons de colorants afin de fournir des signaux SERRS reconnaissables, encapsulés à l'intérieur d'une enveloppe de polymère, ou d'enveloppes.

2. Bille selon la revendication 1, dans laquelle l'enveloppe ou les enveloppes de polymère est/sont formée(s) à partir d'une large variété de monomères et d'agents de réticulation incluant les monomères qui polymérisent via des mécanismes de croissance de chaîne et des mécanismes de croissance par étape capables de former une bille qui encapsule le colloïde agrégé et le colorant actif pour SERRS.

3. Bille selon l'une quelconque des revendications 1 ou 2, dans laquelle ladite enveloppe ou lesdites enveloppes de polymère est/sont formée(s) à partir de n'importe laquelle des classes suivantes et de leurs dérivés: acide acrylique et dérivés incluant l'acide 2-bromoacrylique, le chlorure d'acryloyle, la N-acryloyl tyrosine, la N-acryloyl pyrrolidinone, les acrylates incluant, les acrylates d'alkyle, les acrylates d'allyle, l'acrylate d'hydroxypropyle, l'acide méthacrylique et les dérivés incluant l'acide itaconique, l'acide 2-(trifluorométhyl)propénoïque, les méthacrylates incluant le méthacrylate de méthyle, le méthacrylate d'hydroxyéthyle, le sel sodique de méthacrylate de 3-sulfopropyle, les styrènes incluant le (2,3 et 4)-aminostyrène, l'acide styrène-4-sulfonique, le 3-nitrostyrène, les vinyles incluant le chloroformate de vinyle, l'acide 4-vinylbenzoïque, le 4-vinylbenzaldéhyde, le vinyl imidazole, le 4-vinylphénol, la 4-vinylamine, l'acroléine, les vinylpyridines incluant la (2,3 et 4)-vinylpyridine, le 3-butène 1,2-diol, les acides boroniques incluant l'acide 4-vinylboronique, les acides sulfoniques incluant l'acide 4-vinylsulfonique, les chélateurs de métaux (incluant l'acide styrène iminodiacétique, les acrylamides et les dérivés incluant l'acrylamide de N-méthyle), les méthacrylamides et dérivés incluant le N,N-diméthyl acrylamide, le N-(3-aminopropyl)méthacrylamide, les alcènes-acide 4-penténoïque, l'anhydride d'acide 3-chloro-1-phényl-1-propène (méth)acrylique et les dérivés d'anhydride méthacrylique), les monomères contenant du silicium incluant le (3-méthacryloxypropyl)triméthoxy silane, le tétraméthyldisiloxane, les polyènes-isoprènes, le 3-hydroxy-3,7,11-triméthyl-1,6,10-dodécatriène, les azotures incluant l'acide 4-azido-2,3,5,6-tétrafluorobenzoïque, les thiols incluant l'allyl mercaptan.

4. Bille selon l'une ou l'autre des revendications 1 ou 2, dans laquelle ladite enveloppe ou lesdites enveloppes de polymère est/sont formée(s) à partir d'uréthanes, de carbonates et d'époxy terminés par un acrylate ou sinon insaturés, ou de monomères à base de silicium.

5. Bille selon l'une quelconque des revendications précédentes, dans laquelle ladite enveloppe ou lesdites enveloppes de polymère comprend(comprennent) des agents de réticulation qui fournissent une rigidité aux composés polymères du sujet tels que les acrylates di-, tri-, tétra- et penta-fonctionnels, les méthacrylates, les acrylamides, les vinyles, les allyles, et les styrènes.

6. Bille selon l'une quelconque des revendications précédentes, dans laquelle le colloïde métallique agrégé comprend des surfaces colloïdales à base d'argent, d'or, de cuivre ou d'alliage de métaux.

7. Bille selon l'une quelconque des revendications précédentes, dans laquelle le colloïde métallique agrégé comprend une couche de métal nu ou une couche d'oxyde métallique sur une surface métallique.

8. Bille selon l'une quelconque des revendications précédentes, dans laquelle le colloïde métallique agrégé comprend un revêtement organique de citrate ou d'un polymère polylysine ou polyphénol, pour accroître sa capacité de sorption.

9. Bille selon l'une quelconque des revendications précédentes, dans laquelle le colloïde métallique agrégé se présente sous la forme de particule ou de particules colloïdale(s) métallique(s) agrégée(s).

10. Bille selon la revendication 9, dans laquelle les particules colloïdales métalliques agrégées ont une section transversale de 4 à 50 nm, ou de 25 à 36 nm.

11. Bille selon l'une quelconque des revendications précédentes, dans laquelle la bille a une section transversale inférieure à 1 µm.

12. Bille selon l'une quelconque des revendications précédentes, dans laquelle l'enveloppe de polymère la plus externe comprend en outre des groupes fonctionnels sélectionnés parmi l'un quelconque de ce qui suit: acides carboxyliques, esters actifs d'acides carboxyliques, alcools, amines, époxydes, groupes chlorométhyl styryle, groupes vinyliques, groupes thiol, maléimide et succinimide, ou est en outre dérivatisé à l'aide d'un groupe ou de groupes de liaison, de sorte que les molécules cibles réagissent et deviennent liées à la surface de la bille, ou de sorte qu'une fraction peut réagir et devenir liée à la surface de la bille, laquelle fraction est capable de se lier à ladite molécule cible.

13. Utilisation d'une bille selon l'une quelconque des revendications précédentes, dans la technologie de marquage pour la détection de protéine et d'anticorps.

14. Utilisation d'une bille selon l'une quelconque des revendications précédentes, pour les procédés utilisant l'empreinte moléculaire, dans laquelle lesdites propriétés spécifiques de reconnaissance moléculaire, sont conférées à l'enveloppe de polymère *via* des approches par modèle.

15. Bille selon l'une quelconque des revendications précédentes, comprenant en outre une particule ou des particules magnétique(s) encapsulée(s) à l'intérieur de la bille de polymère, pour magnétiser la bille active pour SERRS.

16. Bibliothèque de billes selon l'une quelconque des revendications précédentes, dans laquelle chaque bille comprend un signal SERRS reconnaissable, le signal SERRS reconnaissable étant obtenu en faisant varier les colorants actifs pour SERRS et/ou les niveaux relatifs des colorants à l'intérieur de chaque bille.

17. Procédé de préparation de billes selon l'une quelconque des revendications précédentes, comprenant les étapes de:
a) formation de nanoparticules d'un colloïde métallique agrégé comprenant un colorant actif pour SERRS absorbé dessus;
b) mélange du colloïde métallique agrégé avec un monomère dans un solvant approprié; et
c) réalisation de la polymérisation du monomère afin de former des billes de polymère encapsulant les nanoparticules du colloïde métallique agrégé ayant absorbé un colorant.

18. Procédé de préparation de billes selon l'une quelconque des revendications précédentes, comprenant les étapes de:
a) fourniture d'un monomère dans un solvant approprié;
b) exécution de la polymérisation du monomère afin de former des billes de polymère;
c) addition des nanoparticules de colloïde métallique agrégé comprenant un colorant actif pour SERRS absorbé dessus à une solution comprenant les billes de polymère; et
d) chauffage de la solution afin que les nanoparticules de colloïde métallique agrégé ayant absorbé un colorant soient prélevées et soient encapsulées par les billes de polymère.

19. Procédé selon la revendication 17 ou 18, dans lequel les billes de polymère sont en outre mises à réagir afin d'ajouter des groupes fonctionnels ou de liaison à une surface externe de la bille permettant ainsi à une molécule d'être fixée à la bille.

20. Utilisation d'une bille ou de billes selon l'une quelconque des revendications 1 à 15, dans un procédé de détection d'une molécule cible dans un échantillon, le procédé comprenant les étapes de:
a) fourniture d'une bille ou de billes selon l'une quelconque des revendications 1 à 15, dans laquelle la bille a été produite afin d'avoir un signal SERRS spécifiquement identifiable et dont la surface a été modifiée afin de présenter fixée dessus une fraction capable de se lier à ladite molécule cible;
b) mise en contact de ladite bille ou desdites billes avec l'échantillon afin de permettre à n'importe laquelle desdites molécules cibles dans l'échantillon de se lier à ladite bille ou auxdites billes; et
c) détection de ladite molécule cible grâce à la détection du signal SERRS spécifiquement identifiable obtenu à partir d'une bille ou de billes qui est(sont) liée(s) à ladite molécule cible.

21. Utilisation selon la revendication 20, dans laquelle la molécule cible est un anticorps, un antigène, un récepteur, un ligand ou une molécule d'acide nucléique, telle qu'une molécule d'ADN.

22. Utilisation selon la revendication 21, dans laquelle la fraction capable de liaison à ladite molécule cible est respectivement un antigène, un anticorps, un ligand, un récepteur ou un acide nucléique complémentaire.

23. Utilisation selon l'une quelconque des revendications 20 à 22, dans laquelle la bille ou les billes sont magnétiques de sorte que la bille ou les billes puisse(nt) facilement être séparée(s) et/ou concentrée(s) après l'étape b).

24. Utilisation d'une bille ou de billes selon l'une quelconque des revendications 1 à 15, dans un procédé de détection d'un acide nucléique spécifique dans un échantillon, comprenant les étapes de:
a) fourniture d'une bille ou de billes selon l'une quelconque des revendications 1 à 15, dans laquelle la bille a été produite afin de présenter un signal SERRS spécifiquement identifiable et dont la surface a été modifiée afin d'avoir, fixée dessus, une fraction capable de liaison à ladite molécule cible dans laquelle la fraction est un oligonucléotide capable de s'hybrider spécifiquement à un acide nucléique cible;
b) mise en contact de ladite bille ou desdites billes avec l'échantillon afin de permettre à n'importe lequel desdits acides nucléiques cibles dans l'échantillon de se lier à ladite bille ou auxdites billes;
c) mise en contact desdites billes liées à la cible avec un autre acide nucléique marqué, ledit acide nucléique marqué devant s'hybrider spécifiquement à celles-ci;
d) conduite éventuellement d'une réaction d'amplification et en outre détection éventuelle du fait que ladite réaction d'amplification a réussi;
e) mise en contact desdites billes liées à un acide nucléique marquées hybridées à une surface capable de liaison à ladite fraction marquée; et
f) détection de ladite molécule cible par la détection d'un signal SERRS spécifiquement identifiable obtenu à partir desdites billes qui ont été effectivement liées audit substrat.

25. Utilisation selon la revendication 24, comprenant en outre une ou plusieurs étape(s) de lavage pour retirer les réactifs non liés des étapes b, c et/ou e à retirer.

26. Utilisation d'une bille ou de billes selon l'une quelconque des revendications 1 à 15, dans un procédé de détection d'une molécule cible dans un échantillon, le procédé comprenant les étapes de:
a) fourniture d'une bille ou de billes actives pour SERRS selon l'une quelconque des revendications 1 à 15, dans laquelle la bille a été produite afin de présenter un signal SERRS spécifiquement identifiable et dont la surface a été modifiée afin d'avoir, fixée dessus, une fraction capable de liaison à ladite molécule cible;
b) fourniture d'une bille magnétique ayant fixé dessus une fraction capable de liaison à ladite molécule cible;
c) mise en contact de ladite bille ou desdites billes et de bille ou de billes magnétique(s) avec l'échantillon afin de permettre à n'importe laquelle desdites molécules cibles dans l'échantillon de se lier à ladite bille ou auxdites billes; et
d) détection de ladite molécule cible par la détection du signal SERRS spécifiquement identifiable obtenu à partir d'une bille ou de billes qui est(sont) liée(s) à ladite molécule cible.

27. Utilisation selon la revendication 26, dans laquelle l'étape c) comprend en outre l'étape de retrait de la bille ou des billes de la solution par centrifugation ou champ(s) magnétique(s).

28. Utilisation d'une bille réactive pour SERRS selon l'une quelconque des revendications 1 à 15, comme marqueur dans un essai de type sandwich.

29. Utilisation selon la revendication 28, dans laquelle un antigène à détecter est piégé par un anticorps immobilisé sur une surface, et un anticorps revêtu sur la bille réactive pour SERRS.

30. Utilisation selon la revendication 29, dans laquelle la surface est celle d'une bille magnétique.

31. Antigène piégé à l'aide d'une bille selon l'une quelconque des revendications 1 à 5, conjointement à un antigène piégé ; et une bille magnétique.
